# EUROPEAN PATENT APPLICATION

(11) **EP 4 529 918 A1**
(43) Date of publication of application: **02.04.2025**
(21) Application number: 24199697.4
(22) Date of filing: 11.09.2024
(51) Int. Cl.: A61K 9/00, A61K 31/465, A61K 47/36, A61P 25/34, A24B 13/00, A61K 47/10, A61K 47/26, A61K 47/32, A61K 47/38

(54) **NICOTINE ORAL FILM AGENT AND PREPARATION METHOD THEREOF**

(30) Priority: 28.09.2023 CN 202311289980
(71) Applicant: Shenzhen Huabao Collaborative Innovation Technology Research Institute Co., Ltd., Shenzhen, Guangdong 518100 (CN)
(72) Inventor: LIU, Yanzi, Shenzhen, Guangdong, 518100 (CN); ZHANG, Han, Shenzhen, Guangdong, 518100 (CN); RONG, Hui, Shenzhen, Guangdong, 518100 (CN); SUN, Qianhui, Shenzhen, Guangdong, 518100 (CN); SHA, Lili, Shenzhen, Guangdong, 518100 (CN)
(74) Representative: Herrero & Asociados, S.L.

(57) **Abstract**

The application discloses a nicotine oral film agent and a preparation method thereof. The nicotine oral film agent comprises a nicotine salt, a film-forming material and an acidity regulator; and the pH of the nicotine oral film agent is 3-6. The nicotine oral film agent prepared by the application has an acidic surface pH, which allows it to moderate the release amount and rate more effectively than those with alkaline or weakly alkaline surfaces. This helps the agent overcome the drawbacks of conventional oral films, such as excessively large nicotine release amounts in a short period of time and too fast nicotine release rate. Meanwhile, the nicotine salt is protonated under acidic conditions, which inhibits the formation of free nicotine, thus avoiding the unstable content of effective components in the film caused by the volatilization of free nicotine and the discoloration caused by the oxidation of free nicotine.

## Description

### Technical field

The application relates to the technical field of oral products, in particular to a nicotine oral film agent and a preparation method thereof.

### Background

A well-known treatment to help quit smoking is to provide smokers with nicotine from sources other than cigarettes. There are various commercially available nicotine replacement products on the market today, including nicotine-containing chewing gum and lozenges, as well as transdermal patches that transfer nicotine into the systemic circulation via mucosal or dermal absorption.

Both nicotine lozenges and nicotine chewing gum contain nicotine bitartrate or nicotine resinate. When chewing gum or swallowing a lozenge, nicotine salt is released from the chewing gum or lozenge and absorbed through the lining of the oral cavity. However, some nicotine will be absorbed with saliva, reducing the amount of nicotine that enters the systemic circulation without first passing through the stomach. The fact that gum or lozenges must be chewed or swallowed for a certain amount of time in order to deliver the entire dose is another drawback. Other evident drawbacks of these means of administration include the fact that chewing gum doesn't always taste good, that it produces waste, and that the resin of gum may even cause cancer in the mouth or throat.

Currently, the commercially accessible tobacco-free products that provide pleasure effects similar to those obtained when smoking are aerosol devices. Nevertheless, there are some disadvantages: users will experience lip numbness and burping. In addition, the aerosol product has an unpleasant flavor, the packing materials in the spray tank are harmful to the environment, and many of the useful ingredients are quickly lost in the surrounding air when spraying.

It is commonly recognized that the pleasant sensation effect in cigarette smoke is created by the protonated nicotine free base present in the smoke. However, nicotine in the form of free base tends to volatile even at temperatures as low as room temperature. Moreover, as a free base, it is likely to be oxidized and photolyzed, and turns brown when exposed to oxygen or air. And, nicotine free base is very hygroscopic, sensitive to moisture, poor in stability and difficult to store.

Orally disintegrating film is an ultra-thin, portable and innovative drug dosage form. After being placed on the recipient's tongue or oral mucosa, the film is soaked in saliva, hydrated and decomposed, and the drug is slowly released and absorbed by the mucosa. This eliminates the need to sip or chew, and the drug is directly absorbed into the blood without being damaged by the liver, thus increasing its bioavailability. The orally disintegrating film is generally composed of pectin, starch, polyvinyl alcohol, sodium alginate, hydroxypropyl methylcellulose and other polymers, supplemented by a plasticizer, flavoring agent and opacifier. Francesco Cilurzo and colleagues developed a low glucose equivalent maltodextrin orally soluble film containing nicotine bitartrate. However, the film made of low glucose equivalent maltodextrin is tougher, less malleable, and decomposes rapidly within 20 seconds, and the drug release is too fast, which is not conducive to the body's absorption and utilization of drugs.

As a new drug delivery dosage form, orally disintegrating film has the advantages of high bioavailability, portability and few adverse reactions, and has huge market potential. However, there are still certain issues with nicotine oral film agents, such as quick nicotine release and insufficient mechanical strength. As a result, one of the most essential research objectives is to develop nicotine oral film agents with a gradual and moderate release amount as well as a steady mechanical strength.

### Summary of the invention

In view of the above shortcomings, the purpose of the present application is to provide a nicotine oral film agent and a preparation method thereof, with the goal of addressing the problems of rapid nicotine release, insufficient mechanical strength, and poor storage stability of the existing nicotine oral film agent.

In order to achieve the above purpose, the application adopts the following technical solutions.

The application provides a nicotine oral film agent, which comprises a nicotine salt, a film-forming material and an acidity regulator; and a pH of the nicotine oral film agent is 3-6.

Preferably, the nicotine salt accounts for 2%-14% of the dry weight of the nicotine oral film agent.

Preferably, the acidity regulator is one or more of hydrochloric acid, citric acid, benzoic acid and acetic acid.

Preferably, the nicotine salt is generated by combining nicotine with an organic acid.

Preferably, the organic acid comprises one or more of benzoic acid, acetic acid, malic acid, tartaric acid, strawberry acid, salicylic acid, lactic acid, levulinic acid, succinic acid, fumaric acid and citric acid.

Preferably, the film-forming material comprises one or more of hydroxypropyl methylcellulose, hydroxypropyl cellulose, sodium carboxymethyl cellulose, microcrystalline cellulose, ethyl cellulose, pullulan, polyvinyl alcohol, polyvinylpyrrolidone, sodium alginate, polyoxyethylene, starch, gelatin, arabic gum, guar gum, pectin and xanthan gum.

Preferably, the nicotine oral film agent further comprises one or more of a solubilizer, a flavoring agent and a plasticizer.

Preferably, the nicotine salt comprises one or more of benzoate, acetate, malate, tartrate, bitartrate, strawberry acid, salicylate, lactate, levulinic acid, succinate, fumarate and citrate; preferably benzoate, malate, tartrate and bitartrate.

Preferably, the solubilizer comprises polysorbate, sodium dodecyl benzene sulfonate, lecithin, magnesium stearate, benzalkonium chloride; preferably polysorbate and sodium dodecyl benzene sulfonate.

Preferably, the plasticizer comprises polyethylene glycol (PEG), glycerol or propylene glycol.

Preferably, the flavoring agent comprises one or more of sweetener, aromatic agent and cooling agent.

The thickness of the film plays an important role. If it is too thick, it will cause discomfort in the mouth, and if it is too thin, it will be inconvenient to take and use. Preferably, the thickness of the nicotine oral film agent is 0.01-0.3 mm. This thickness is suitable and brings a certain strength to facilitate use.

The application further provides a preparation method of the nicotine oral film agent, which comprises the following steps:
S1: mixing a nicotine salt, a solubilizer, a flavoring agent, a plasticizer and water to form a mixture;
S2: adjusting the pH of the mixture to 3-6.5 by using an acidity regulator to obtain an acidic solution;
S3: adding a film-forming material into the acidic solution, and stirring until a viscosity of the acidic solution is 8000-50000 mpa.s to obtain a film-forming solution; and
S4: coating the film-forming solution on a substrate, and drying to obtain the nicotine oral film agent.

Preferably, in step S4, the substrate is a plastic substrate; the drying temperature is 50-80°C, and the drying time is 15-40 min.

The application has the following beneficial effects.

The nicotine oral film agent prepared by the application has an acidic surface pH, which allows it to moderate the release amount and rate more effectively than those agents with alkaline or weakly alkaline surfaces. This helps the agent overcome the drawbacks of conventional oral films, such as excessively large nicotine release amounts in a short period of time and too fast nicotine release rate. Meanwhile, the nicotine salt is protonated under acidic conditions, which inhibits the formation of free nicotine, thus avoiding the unstable content of effective components in the film caused by the volatilization of free nicotine and the discoloration of the film caused by the oxidation of free nicotine.

### Brief description of the drawings

The attached drawings will be briefly described below in order to help better understand the technical solutions disclosed in the following embodiments of the current application.
Fig. 1 shows the appearance of the nicotine oral film agent after being stored for 180 days in an open environment with room temperature and relative humidity of 20%-60%, according to Embodiment 1 and Comparative Example 1.
Fig. 2 is a graph of nicotine release from the nicotine oral film agent prepared according to Embodiment 1.
Fig. 3 is a graph of nicotine release from the nicotine oral film agent prepared according to Comparative Example 1.

### Detailed description of disclosed embodiments

The technical solutions in the embodiments of the present application will be clearly and completely described below with reference to the drawings in the embodiments of this application. Obviously, the described embodiments are merely part of the embodiments of this application, not all of them. Based on the embodiments in this application, all other embodiments obtained by those of ordinary skill in the art without creative effort belong to the protection scope of this application.

The embodiment of the application provides a nicotine oral film agent, which comprises a nicotine salt, a film-forming material and an acidity regulator; and the pH of the nicotine oral film agent is 3-6.

The acidity regulator may be one or more of hydrochloric acid, citric acid, benzoic acid and acetic acid. The film-forming material includes one or more of hydroxypropyl methylcellulose, hydroxypropyl cellulose, sodium carboxymethyl cellulose, microcrystalline cellulose, ethyl cellulose, pullulan, polyvinyl alcohol, polyvinylpyrrolidone, sodium alginate, polyoxyethylene, starch, gelatin, arabic gum, guar gum, pectin and xanthan gum. The nicotine salt may be a pharmaceutically acceptable salt, such as one or more of benzoate, acetate, malate, tartrate, bitartrate, strawberry acid, salicylate, lactate, levulinic acid, succinate, fumarate and citrate. Preferably benzoate, malate, tartrate and bitartrate.

In some embodiments of the present application, the nicotine salt accounts for 2%-14% of the dry weight of the nicotine oral film agent, such as 2%, 3%, 5%, 8%, 10%, 14%, etc.

In some embodiments of the application, a solubilizer, flavoring agent, plasticizer or other auxiliary material may also be added to the nicotine oral film agent according to actual needs. The solubilizer may be polysorbate, sodium dodecyl benzene sulfonate, lecithin, magnesium stearate, benzalkonium chloride, etc. Preferably polysorbate and sodium dodecyl benzene sulfonate.

The plasticizer may be polyethylene glycol (PEG), glycerol or propylene glycol. The flavoring agent may be one or more of sweetener, aromatic agent and cooling agent. The sweetener includes saccharin sodium, acesulfame K, sucralose, neotame, aspartame, stevioside, siraitia grosvenorii, mannitol, sucrose, etc. the aromatic agent includes but is not limited to any natural or synthetic essence, eucalyptus oil, sweet orange oil, mint (such as peppermint and Dutch mint), chocolate, licorice, citrus plants and other fruit flavors, γ-Octalactone, vanillin, ethyl vanillin, flavors such as cinnamon, methyl salicylate, linalool, bergamot oil, geranium oil, lemon oil and ginger oil. The cooling agent includes one or two of WS-3(N- ethyl -2- isopropyl -5- methyl-cyclohexane formamide) and WS-23(N,2,3- trimethyl -2-isopropyl butyramide).- The other auxiliary material includes a pigment, an antioxidant or a preservative.

In some embodiments, the pH of the film-forming solution is adjusted to 3-6.5, 4-6, or 4.5-5 by using an acidity regulator to prepare a dry film with a pH of 3-6. For example, the dry film has a pH of 6, 5.5, 5, 4.5, 4, 3.5 or 3.

In some embodiments, the dry film agent prepared by the present application has a thickness of 0.01 mm-0.3 mm. For example, 0.02 mm-0.2 mm, 0.03 mm-0.15 mm or 0.08 mm.

In some embodiments, the preparation method of the nicotine oral film agent comprises the following steps:
S1: mixing a nicotine salt, a solubilizer, a flavoring agent, a plasticizer and water to form a mixture;
S2: adjusting the pH of the mixture to 3-6.5 by using an acidity regulator to obtain an acidic solution;
S3: adding a film-forming material into the acidic solution, and stirring until a viscosity of the acidic solution is 8000-50000 mpa.s to obtain a film-forming solution; and
S4: coating the film-forming solution on a substrate, and drying to obtain the nicotine oral film agent.

Here, a coating method (doctor-blade process) may be used to prepare the dry film. For example, the film-forming solution containing nicotine is applied onto a plastic film to generate a wet film with a thickness of 0.3-1.5 mm, and then the wet film is dried in a ventilated oven at 50°C-80°C for 15-40 min or 20-30 min. After the film has completely dried, the resulting dry film (thickness of 0.01-0.3 mm) is cut into dose units of acceptable size and placed in a suitable storage container. The storage container could be a sealed PET box or a polyester/aluminum/polyethylene composite film.

The nicotine oral film agent of the present application may have any acceptable surface area. For example, a dose unit with a surface area of 3cm², in a determined dose unit. The acceptable dose units include, for example, 0.5-8 mg of nicotine (2%-14%), or 1-2 mg of nicotine, or any other suitable amount. For example, the unit dose may have a surface area of 3cm², a thickness of 0.08 mm, and a dry film containing 2 mg of nicotine. The nicotine oral film agent may have any suitable shapes, such as rectangle, rounded rectangle, ellipse, round, etc.

Compared with the prior art, the application has the following beneficial effects.
1. The content of nicotine in the film agent of the present application is stable. The stability of this content depends on the acidic environment of the nicotine film-forming solution. After the nicotine salt is dissolved in aqueous solution, the protonated nicotine and non-protonated nicotine are in a dynamic equilibrium state, which is greatly influenced by pH value.
The hydrolysis process of nicotine under different pH conditions is as follows: 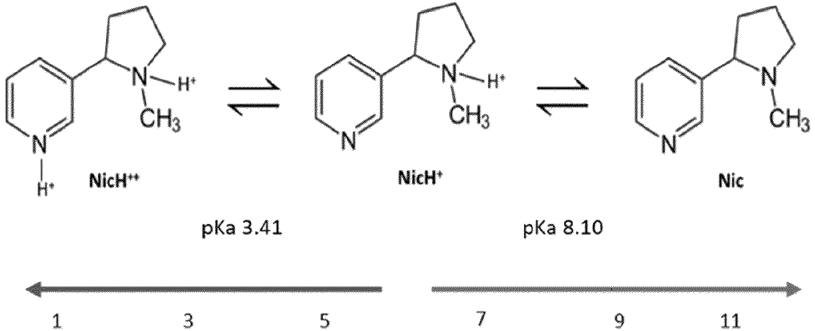 when the pH of the solution is less than 7, the nicotine in the solution exists in protonated form, which inhibits the generation of free nicotine. In order to prevent nicotine loss due to the production and volatilization of free nicotine, the pH of the nicotine film-forming solution is adjusted to be acidic during the preparation process. This ensures the stability of the nicotine content.
2. The nicotine film of the application is well-maintained, and discoloration due to oxidation hardly happens throughout the course of the shelf life. The reason for this is that the nicotine film-forming solution is acidic, the nicotine in the film-forming solution basically exists in protonated form, and the nicotine in the dry film formed after drying also exists in protonated form, with little free nicotine, thereby preventing the film from turning brown due to oxidation of free nicotine.
3. The nicotine released from the oral film agent of the present application is moderate. The absorption of nicotine through oral mucosa is pH-dependent, and the nicotine salt and acidifier are released when the film dissolves. With saliva at the oral mucosa adhered to the oral film agent, the acidifier will create an acidic environment with a pH of 3-6. When the pH is preferably less than or equal to 5.5, nicotine would almost entirely exist in protonated form and permeates into the bloodstream through the cell bypass route, thereby realizing the systemic delivery of nicotine. Compared with free-radical nicotine, the protonated form of nicotine is released relatively slowly and produces a smoother sensory effect.
Table 1 shows the relative amounts of protonated and non-protonated nicotine at three pH values at 37°C. It is evident that nicotine basically exists in protonated form when the pH value of the film material containing nicotine is 3-7, and 99.6% of nicotine in the solution is protonated when the pH value is ≤ 5.5.

**Table 1**

| | NH+NH+ | NNH+ | NN |
|---|---|---|---|
| PH 5.5 | 0.6 | 99.0 | 0.4 |
| PH 7.4 | 0 | 75.8 | 24.2 |
| PH 8.1 | 0 | 38.5 | 61.5 |

4. The nicotine oral film agent of the present application has good mechanical strength, which ensures a proper thickness and also has a certain strength for ease of usage.

In summary, the application prevents the generation of nicotine by regulating the acidity of the nicotine oral film. This puts the nicotine in a protonated state, which enables it to moderate the release amount and rate. This prevents the oxidative discoloration of the film materials and unstable content issues brought on by the volatilization of free nicotine. When users utilize the application's nicotine oral film agent, it dissolves in the presence of saliva and releases protonated nicotine. When protonated nicotine permeates the oral mucosa and enters into the bloodstream, it produces feelings of pleasure and contentment. The nicotine oral film agent of the present application is in the form of a film adhered to oral mucosa. When applied to the oral mucosa, the dry film will adhere to it and dissolve in a certain time. For example, 1-30 min, or 1-20 min, or 1-5 min. The nicotine salt and acidifier are released when the film is dissolved. With saliva at the oral mucosa adhered to the oral film agent, the acidifier will create an acidic environment with a pH of 3-6. In this acidic liquid solution, nicotine would exist in protonated form and permeates into the bloodstream through the cell bypass route, thereby realizing the systemic delivery of nicotine.

The technical solution and effect of the present application will be described in detail through specific embodiments.

### Embodiment 1

Nicotine-containing oral film was prepared using the materials listed in Table 2.

**Table 2**

| Material | Dosage |
|---|---|
| Nicotine tartrate | 5g |
| Polyvinylpyrrolidone (PVP) | 6g |
| Hypromellose | 28g |
| Polyethyleneglycol | 7g |
| Sucralose | 0.4g |
| Strawberry essence | 1g |
| Tween 80 | 4g |
| Purified water | 200g |
| pH adjusted with HCl | 6.0 |

The preparation method of the nicotine oral film agent comprises the following steps.
S1: placing nicotine tartrate in a suitable container, adding purified water for dissolution, then adding solubilizer, flavoring agent and plasticizer, and uniformly stirring to produce a mixture;
S2: adjusting the pH of the mixture to 6.0 by using HCl to obtain an acidic solution;
S3: adding a film-forming material into the acidic solution, stirring at a rotating speed of 1000r/min at room temperature to obtain a uniform and continuous film-forming solution, and measuring the viscosity of the film-forming solution at a shearing rate of 6 rpm using a Brinell viscometer with a No.4 rotor until the viscosity of the film-forming solution is 8,000-50,000 MPa. s;
S4: after removing bubbles with vacuum centrifugation for 30 min, applying the film-forming solution onto a plastic film to obtain a wet film, and then drying the wet film in a ventilated oven at 70°C for 30min. To acquire the nicotine oral film agent, removing the dry film from the plastic film, cut it into 1.5cm×2cm rectangular blocks, and package them separately.

### Embodiment 2

Nicotine-containing oral film was prepared using the materials listed in Table 3.

**Table 3**

| Material | Dosage |
|---|---|
| Nicotine tartrate | 12g |
| Polyvinylpyrrolidone (PVP) | 6g |
| Hypromellose | 28g |
| Polyethyleneglycol | 7g |
| Sucralose | 0.4g |
| Strawberry essence | 1g |
| Tween 80 | 4g |
| Purified water | 200g |
| Adjust pH with HCl | 4.5 |

The preparation method of the nicotine oral film agent comprises the following steps.
S1: placing nicotine tartrate in a suitable container, adding purified water for dissolution, then adding solubilizer, flavoring agent and plasticizer, and uniformly stirring to produce a mixture;
S2: adjusting the pH of the mixture to 4.5 by using HCl to obtain an acidic solution;
S3: adding a film-forming material into the acidic solution, stirring at a rotating speed of 1000r/min at room temperature to obtain a uniform and continuous film-forming solution, and measuring the viscosity of the film-forming solution at a shearing rate of 6 rpm using a Brinell viscometer with a No.4 rotor until the viscosity of the film-forming solution is 8,000-50,000 MPa. s;
S4: after removing bubbles with vacuum centrifugation for 30 min, applying the film-forming solution onto a plastic film to obtain a wet film, and then drying the wet film in a ventilated oven at 70°C for 30min. To acquire the nicotine oral film agent, removing the dry film from the plastic film, cut it into 1.5cm×2cm rectangular blocks, and package them separately.

### Embodiment 3

Nicotine-containing oral film was prepared using the materials listed in Table 4.

**Table 4**

| Material | Dosage |
|---|---|
| Nicotine tartrate | 5g |
| Polyoxyethylene | 24g |
| Hypromellose | 10g |
| Glycerol | 8g |
| Aspartame | 0.8g |
| Strawberry essence | 1g |
| Tween 80 | 4g |
| Purified water | 200g |
| Adjust pH with HCl | 3.0 |

The preparation method is the same as in Embodiment 1.

### Embodiment 4

Nicotine-containing oral film was prepared using the materials listed in Table 5.

**Table 5**

| Material | Dosage |
|---|---|
| Nicotine tartrate | 5g |
| Polyoxyethylene | 24g |
| Hypromellose | 10g |
| Propylene glycol | 10g |
| Aspartame | 0.8g |
| Mint essence | 1g |
| Tween 80 | 4g |
| Purified water | 200g |
| Adjust pH with HCl | 4.0 |

The preparation method is the same as in Embodiment 1.

### Comparative Example 1

Nicotine-containing oral film was prepared using the materials listed in Table 6.

**Table 6**

| Material | Dosage |
|---|---|
| Nicotine tartrate | 5g |
| Polyvinylpyrrolidone (PVP) | 6g |
| Hypromellose | 28g |
| Polyethyleneglycol | 7g |
| Sucralose | 0.4g |
| Strawberry essence | 1g |
| Tween 80 | 4g |
| Purified water | 200g |
| Adjust pH sodium bicarbonate. | 11.5 |

The preparation method is as follows.

The preparation method of the nicotine oral film agent comprises the following steps.
S1: placing nicotine tartrate in a suitable container, adding purified water for dissolution, then adding solubilizer, flavoring agent and plasticizer, and uniformly stirring to produce a mixture;
S2: adjusting the pH of the mixture to 11.5 using sodium bicarbonate to obtain an alkaline solution;
S3: adding a film-forming material into the alkaline solution, stirring at a rotating speed of 1,000 r/min at room temperature to obtain a uniform and continuous film-forming solution, and measuring the viscosity of the film-forming solution at a shearing rate of 6 rpm using a Brinell viscometer with a No.4 rotor until the viscosity of the film-forming solution is 8,000-50,000 MPa. s;
S4: after removing bubbles with vacuum centrifugation for 30 min, applying the film-forming solution onto a plastic film to obtain a wet film, and then drying the wet film in a ventilated oven at 70°C for 30min. To acquire the nicotine oral film agent, removing the dry film from the plastic film, cut it into 1.5cm×2cm rectangular blocks, and package them separately.

### Performance test

### 1. Stability of nicotine content in the nicotine oral film agent.

The dose units prepared in Embodiment 1 and Embodiment 2 were used for this test. On the 0 th day of the study, the package filled with dry film was unsealed, and the nicotine concentration in one dose unit (i. e., a piece of oral film with an area of 3 cm², a thickness of 0.08mm and a weight of 35 mg) was measured. The remaining dose units contained in the package were stored in an open environment with a temperature of 10-40°C and a relative humidity of 20%-60%. The nicotine concentrations of the two dose units were measured again on the 30th, 90th and 180th day.

Specifically, the nicotine concentration testing method comprises the following steps.

Pretreatment: dissolve the film in 5mL of water, add methanol to 10mL, and prepare for injection after filtering film.
Instruments: Agilent HPLC 1260, UV detector.
Chromatographic column: C18 column (250mm×4.6nm, 5µm)
Column temperature: 40°C
Mobile phase: methanol with volume ratio of 40:60-20 mM phosphate buffer containing 0.2% triethylamine (pH=6).
Flow rate: 1 mL/min
Detection wavelength: 259 nm
Draw a standard curve of nicotine hydrogen tartrate dihydrate.

The test results are shown in Table 7.

**Table 7**

| Storage days | Nicotine content (mg)/ dose unit | |
|---|---|---|
| | Embodiment 1 | Embodiment 2 |
| 0 | 1.856 | 3.723 |
| 30 | 1.849 | 3.720 |
| 90 | 1.853 | 3.718 |
| 180 | 1.850 | 3.725 |

As can be seen from Table 7, the effective materials in the nicotine oral film agent provided by the application remain unchanged after 180 days of storage, and the shelf life is long.

### 2. Mechanical properties of the nicotine oral film agent

The mechanical properties of the nicotine oral film agents prepared in Embodiments 1 to 4 were investigated, and the results are shown in Table 8.

**Table 8 Comparison of Mechanical Properties of the nicotine oral film agents prepared in different Embodiments**

| Sample | Tensile strength TS (Mpa) | Elongation at break EB(%) |
|---|---|---|
| Embodiment 1 | 14.25±2.58 | 19.47±2.87 |
| Embodiment 2 | 14.55±2.17 | 20.22±2.43 |
| Embodiment 3 | 20.87±3.41 | 26.21±3.14 |
| Embodiment 4 | 18.58±2.59 | 24.24±3.48 |

It can be seen from the above table that the film agent obtained by the embodiments of the application has good mechanical properties.

### 3. pH and stability of the nicotine oral film agent

### 3.1 Tests for the surface pH of the films prepared in Embodiment 1 and Comparative Example 1 respectively.

The test results are shown in Table 9.

**Table 9**

| Sample | Surface pH |
|---|---|
| Embodiment 1 | 5.5 |
| Comparative Example 1 | 11.3 |

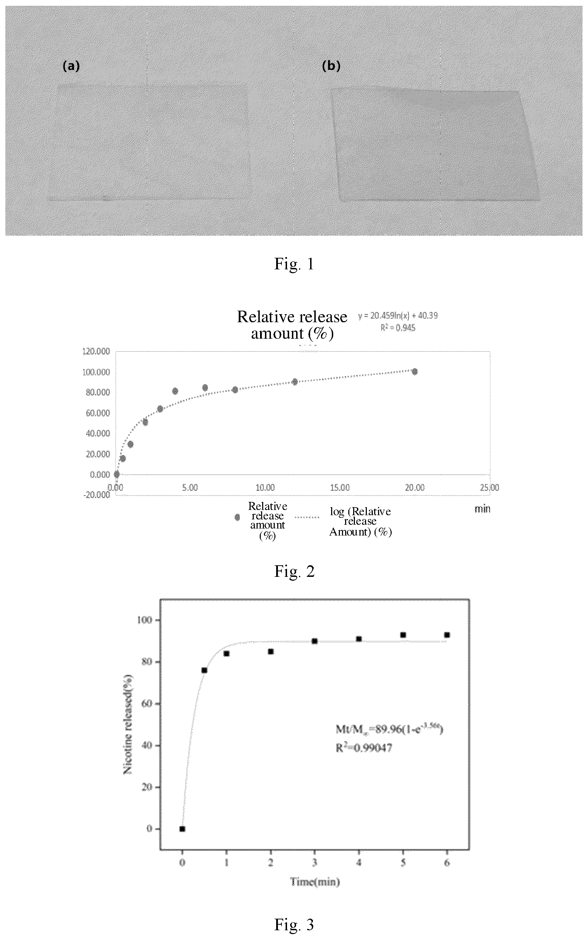

3.2 Embodiment 1 and Comparative Example 1 were stored in an open environment with room temperature and relative humidity of 20%-60% for 180 days, and the results are shown in Figure 1. It can be seen that the film of Comparative Example 1 (Figure 1b) turned brown after 180 days, and the appearance was not well maintained, while Embodiment 1 (Figure 1a) did not have obvious appearance changes after 180 days of storage. Therefore, the appearance of the dry film formed by drying the acidic nicotine film-forming solution remains good.

### 4. Experiment of nicotine release from the nicotine oral film agent.

200 ml of simulated artificial saliva with pH 6.8 was put into each of the two beakers, and kept at 37.0 0.1°C and stirred at 100 rpm. The films of Embodiment 1 and Comparative Example 1 were put into two beakers respectively. For 10 min, 2mL was precisely sampled every minute, and then 2mL of simulated artificial saliva was added to the sample. The 2 mL solution was adjusted to 10 mL with simulated artificial saliva, and the nicotine content was detected by HPLC to calculate the relative cumulative release of nicotine. The release curve was plotted with the relative cumulative release as the ordinate and time as the abscissa.

As shown in Figures 2-3, compared with the nicotine oral film agent with alkaline surface, the acidic nicotine oral film agent prepared by the embodiment of the present application allows it to moderate the release amount and rate. This helps the agent overcome the drawbacks of conventional oral films, such as excessively large nicotine release amounts in a short period of time and too fast nicotine release rate.

It should be noted that all of the foregoing embodiments are based on the same innovative concept, and each embodiment's description emphasizes its own aspect. Please see the descriptions of other embodiments for more information on the specifics of each.

The preceding embodiment just indicates the implementation of the application, and its description is more specific and detailed, but it cannot be interpreted as restricting the scope of the application patent. It should be noted that for those skilled in the art, a number of alterations and improvements could be made without departing from the concept of the invention, all of which are within the protection scope of the application. Therefore, the protection scope of the present application should be based on the appended claims.

## Claims

1. A nicotine oral film agent, **characterized by** comprising a nicotine salt, a film-forming material and an acidity regulator; and a pH of the nicotine oral film agent is 3-6.

2. The nicotine oral film agent of claim 1, wherein the nicotine salt accounts for 2%-14% of the dry weight of the nicotine oral film agent.

3. The nicotine oral film agent of claim 1, wherein the thickness of the nicotine oral film agent is 0.01-0.3 mm.

4. The nicotine oral film agent of claim 1, wherein the acidity regulator is one or more of hydrochloric acid, citric acid, benzoic acid and acetic acid.

5. The nicotine oral film agent of claim 1, wherein the nicotine salt is generated by combining nicotine with an organic acid.

6. The nicotine oral film agent of claim 5, wherein the organic acid comprises one or more of benzoic acid, acetic acid, malic acid, tartaric acid, strawberry acid, salicylic acid, lactic acid, levulinic acid, succinic acid, fumaric acid and citric acid.

7. The nicotine oral film agent of claim 1, wherein the film-forming material comprises one or more of hydroxypropyl methylcellulose, hydroxypropyl cellulose, sodium carboxymethyl cellulose, microcrystalline cellulose, ethyl cellulose, pullulan, polyvinyl alcohol, polyvinylpyrrolidone, sodium alginate, polyoxyethylene, starch, gelatin, arabic gum, guar gum, pectin and xanthan gum.

8. The nicotine oral film agent of claim 1, further comprising one or more of a solubilizer, a flavoring agent and a plasticizer.

9. A preparation method of the nicotine oral film agent according to any one of claims 1 to 8, **characterized by** comprising the following steps:
S1: mixing a nicotine salt, a solubilizer, a flavoring agent, a plasticizer and water to form a mixture;
S2: adjusting the pH of the mixture to 3-6.5 by using an acidity regulator to obtain an acidic solution;
S3: adding a film-forming material into the acidic solution, and stirring until a viscosity of the acidic solution is 8000-50000 mpa.s to obtain a film-forming solution; and
S4: coating the film-forming solution on a substrate, and drying to obtain the nicotine oral film agent.

10. The preparation method of the nicotine oral film agent of claim 9, wherein in step S4, the substrate is a plastic substrate; the drying temperature is 50-80°C, and the drying time is 15-40 min.

11. The nicotine oral film agent of claim 1, the nicotine salt comprises one or more of benzoate, acetate, malate, tartrate, bitartrate, strawberry acid, salicylate, lactate, levulinic acid, succinate, fumarate and citrate; preferably benzoate, malate, tartrate and bitartrate.

12. The nicotine oral film agent of claim 8, the solubilizer comprises polysorbate, sodium dodecyl benzene sulfonate, lecithin, magnesium stearate, benzalkonium chloride; preferably polysorbate and sodium dodecyl benzene sulfonate.

13. The nicotine oral film agent of claim 8, the plasticizer comprises polyethylene glycol (PEG), glycerol or propylene glycol.

14. The nicotine oral film agent of claim 8, the flavoring agent comprises one or more of sweetener, aromatic agent and cooling agent.
